# EUROPEAN PATENT APPLICATION

(11) **EP 3 714 851 A1**
(43) Date of publication of application: **30.09.2020**
(21) Application number: 19166328.5
(22) Date of filing: 29.03.2019
(51) Int. Cl.: A61F 13/15, A61F 13/62, A61F 13/56

(54) **DIAPER FASTENING TAB, DIAPER AND METHOD OF MAKING THE SAME**

(71) Applicant: 3M Innovative Properties Company, Saint Paul, MN 55133-3427 (US)
(72) Inventor: Hauschildt, Mr. Volker, 40723 Hilden (DE); Genç, Mr. Yavuz, Corlu/Tekirdag (TR); Peiffer, Mr. Andreas, Neuss 41470 (DE)
(74) Representative: Vollmers, Hans-Gerd

(57) **Abstract**

The present disclosure relates to a diaper fastening tab 100 comprising a user's end 70 to be gripped by a user, an opposite manufacturer's end 80, a first and a second major surface, a user's end portion 50 adjacent to the user's end 70, a manufacturer's end portion 60 adjacent to the manufacturer's end 80, wherein the user's end portion 50 comprises on the first major surface a first and at least one second strip 30, 40 of mechanical fastening elements 32, 42 each with a different peel strength as measured according to ASTM D 5170-98, wherein a first strip 30 with a higher peel strength is located towards the user's end 70 and at least one second strip 40 with a lower peel strength is located towards the manufacturer's end 80. The present disclosure further relates to a roll 110 of diaper fastening tape 120 from which such diaper fastening tabs 100 can be obtained as well as to a diaper 150 comprising such a diaper fastening tab 100. The present disclosure moreover relates to a method of making such a diaper fastening tab 100 as well as to a method of making a diaper 150 with such diaper fastening tabs 100.

## Description

The present disclosure relates to a diaper fastening tab which is suitable for reliably preventing unintentional disengagement from fibrous materials. The present disclosure also relates to a roll of diaper fastening tape from which such diaper fastening tabs can be obtained. The present disclosure furthermore relates to methods of making such a fastening tab as well as a diaper with such fastening tabs.

Mechanical fasteners, which are also called hook and loop fasteners, are useful for providing releasable attachment in numerous applications. For example, mechanical fasteners are widely used in wearable absorbent articles to fasten such articles around the body of a person. In typical configurations, a hook strip or patch on a fastening tab attached to the rear waist portion of a diaper or incontinence garment, for example, can fasten to a landing zone of loop material on the front waist region, or the hook strip or patch can fasten to the backsheet (e. g., nonwoven backsheet) of the diaper or incontinence garment in the front waist region. Absorbent articles often employ woven or nonwoven materials, for example, to provide a cloth-like feeling in order to increase the comfort of wearing.

Fastening tabs often have a manufacturer's end that is attached to the rear waist region of an absorbent article and a user's end that can be grasped and extends outwardly beyond the edge of an absorbent article before it is attached to the front waist region of the absorbent article. The attachment point of the tab at the manufacturer's end must be strong enough to withstand the force applied during the application and wearing of the absorbent article; otherwise the tab can separate from the absorbent article during use.

Some fastening tabs have more than one region of mechanical fastener (e. g., hook patches) on the tab, which has been proposed to decrease the likelihood of unintentional disengagement of the mechanical fastener members. See, for example, U. S. Pat. Nos. 5,957,908 (Kline et al.) and 5,851,205 (Hisada et al.).

Fastening tabs with hook strips are, for example, described in WO-A-2005/000180 (3M Innovative Properties), EP-B-1725201 (Koester) and others. The hook material is separated into two or more hook strips in order to further decrease the likelihood of unintentional disengagement of the mechanical fastener members.

Although several solutions exist to address the problem of unintentional disengagement of the mechanical fastener, there is still a need to further improve the prevention of such unintentional disengagement.

It is therefore an object of the present disclosure to provide a diaper fastening tab with a mechanical fastener which is capable to prevent unintentional disengagement of the mechanical fastener to a sufficient extent.

The present disclosure relates to a diaper fastening tab comprising a user's end to be gripped by a user and an opposite manufacturer's end. The diaper fastening tab comprises a first and a second major surface. The diaper fastening tab further comprises a user's end portion adjacent to the user's end and a manufacturer's end portion adjacent to the manufacturer's end. The user's end portion comprises on the first major surface a first and at least a second strip of mechanical fastening elements each with a different peel strength as measured according to ASTM D 5170-98. The first and the at least one second strips may be discrete strips. The first strip with a higher peel strength is located towards the user's end and the at least one second strip with a lower peel strength is located towards the manufacturer's end. The term "end" denotes an edge of the diaper fastening tab, whereas the term "portion" denotes an area on the first surface of the diaper fastening tab.

The diaper fastening tab may comprise a carrier. The carrier may comprise a first and a second major surface. The first and the at least one second strips may be arranged on the first major surface. The carrier may be made of a thermoplastic material, for example polyethylene or polypropylene. The carrier may exhibit a thickness of 10 to 1000 µm, preferably 50 to 500 µm. The carrier may exhibit an extension in machine direction of 10 - 70 mm and an extension in cross-direction of 30 - 100 mm. The extension of the user's end portion 50 measured in cross-direction is typically 15 - 50 mm.

The term "machine direction" (MD) as used above and below denotes the direction of the running, continuous web of the diaper fastening tape, from which individual diaper fastening tabs can be cut, during the manufacturing. In case of a roll, the machine direction is the direction of unwinding the roll. In case of a diaper fastening tab, the machine direction is the direction from one longitudinal side to the other longitudinal side edge. In other words, the machine direction is the direction along the transverse edges of the diaper fastening tab. The term "cross direction" (CD) is the direction of the running, continuous web, which is essentially normal to the machine direction. The cross direction of the diaper fastening tab is the direction normal to the machine direction. These terms will also be described in more detail with reference to the drawings.

The term "strip" as used above and below denotes an elongated or square-shaped piece of material comprising mechanical fastening elements. The strip may comprise a backing. The backing may comprise only one material and exhibit an essentially uniform construction in CD, but it may also comprise a sequence of two or more zones in CD having different properties whereby such zones preferably extend continuously in MD. The backing may, for example, be manufactured in a way that layers of different materials are co-extruded or laminated to each other.

The first strip provides higher resistance against peel forces and helps to prevent unintentional disengagement of the fastening tab from a fibrous material or from a female fastening material, respectively. This means that even with at least one second strip having a lower resistance against peel forces, unintentional disengagement is reliably prevented as the disengagement typically starts from the user's end, where a higher peel resistance is provided. The solution according to the present disclosure also provides for a cost-efficient diaper fastening tab, because using a second strip with a lower peel strength is typically more cost-effective as this material is cheaper than the material of the first strip.

In one embodiment, the manufacturer's end portion is free of mechanical fastening elements. This is advantageous because there are no mechanical fastening elements in an area where the diaper fastening tab is attached to the diaper, e. g. by means of adhesive, thermal bonding like for example ultrasonic bonding, hot air bonding or laser welding. It is also cost saving, since no additional mechanical fastening elements need to be provided.

In another embodiment, the user's end portion and the manufacturer's end portion abut each other forming a separation line on the diaper fastening tab. Such a solution would be beneficial as it provides for a cost-efficient, simple and space-saving solution. Alternatively, a further portion is arranged between the manufacturer's end portion and the user's end portion, for example an elastic portion. In this case, the manufacturer's end portion and the user's end portion do not abut each other. Such a further portion may be beneficial as it provides for further functionality of the diaper fastening tab, for example it would render the diaper fastening tab elastic.

In one embodiment, the user's end portion and the manufacturer's end portion each have a width in cross-direction of the diaper fastening tab. Each portion has a width of 50 % of the width in cross-direction of the diaper fastening tab. Such a distribution of the manufacturer's end portion and the user's end portion on the diaper fastening tab may be beneficial because enough area is provided for the fastening elements on the one hand and enough area is provided for attaching and securely bonding the diaper fastening tab to a diaper on the other hand. Alternatively, the width of the user's end portion may be more than 50 % of the width of the diaper fastening tab in cross direction. In this case, there is an enlarged portion provided which, for example, allows for arranging more strips of mechanical fastening elements in the user's end portion. As further alternative, the width of the manufacturer's end may be more than 50% of the width of the diaper fastening tab in cross direction. With such a solution, an increased area for attaching and bonding of the diaper fastening tab to a diaper is provided which may help to increase the bonding security of the diaper fastening tab to the diaper.

In one embodiment, each of the first and at least one second strips of mechanical fastening elements comprise a separate backing from which the mechanical fastening elements protrude. This is advantageous because such an arrangement provides for a high flexibility of the diaper fastening tab. Also, the material of the first strip and of the second strip can be selected separately and manufactured separately and can then be applied by unwinding the two materials from different supply rolls. This provides for an easy and reliable manufacture of the diaper fastening tab. It also provides for a cost-effective solution, because the selection of the material may be adopted to the respective needs of each strip.

The first strip with a higher peel strength comprises in one embodiment the mechanical fastening elements exhibiting a larger height compared to the height of the mechanical fastening elements of the at least one second strip with a lower peel strength. The height is measured in a direction normal to the machine direction and the cross direction. This is beneficial because mechanical fastening elements with a larger height reliably provide for higher peel strength. This is one example of providing a first strip of mechanical fastening elements with a higher peel strength next to a second strip of mechanical fastening elements with lower peel strength.

In one embodiment, the first strip with a higher peel strength comprises a backing exhibiting a greater thickness compared to the thickness of the backing of the at least one second strip with a lower peel strength. The thickness is measured in a direction normal to the machine direction and the cross direction. This is beneficial because a strip of mechanical fastening elements with a greater thickness reliably provides for higher peel strength. This is another example of providing a first strip of mechanical fastening elements with a higher peel strength next to a second strip of mechanical fastening elements with lower peel strength.

The mechanical fastening elements of the first strip and/or of the at least on second strip may in one embodiment comprise a stem projecting from a major surface of the backing with an enlarged section which is positioned at the end of the stem opposite of the surface of the backing. Such stems preferably are essentially cylindrical, oval or rectangular cross-sectional shape. The enlarged section of the mechanical fastening elements may have any shape such as hooks, T's, J's, mushroom-type heads (including concavely curved heads or disc-shaped heads) or any other shape allowing for engagement with complementary female fastening elements. This is advantageous because mechanical fastening elements with an enlarged section typically exhibit an increased engageability and thus further help to increase the peel strength of the mechanical fastening elements. It is possible that the mechanical fastening elements of both strips comprise enlarged sections which are positioned at the end of the stem or only one of them. Providing both strips with mechanical fastening elements with enlarged sections may even further help to increase the peel strength of the mechanical fastening elements. Alternatively, the mechanical fastening elements can also be formed by stems having no enlarged section at the end of the stem opposite to the backing wherein such stems preferably are essentially cylindrical, oval or rectangular cross-sectional shape.

In one embodiment, the mechanical fastening elements are uniformly shaped within one strip. Such a uniform shape within one strip is advantageous because such a strip can be manufactured in an easy, cost-efficient and reliable way. The mechanical fastening elements may also be uniformly shaped within the first strip on the one hand and within the at least one second strip on the other hand. This may further help to manufacture the diaper fastening tab in an easy, cost-efficient and reliable way.

In one embodiment, the first and the at least one second strips are attached to the first major side of the fastening tab by an adhesive layer. Adhesives are beneficial because they provide for an easy to apply, cost-efficient and reliable way of attachment of such strips of mechanical fastening elements. Alternatively, the strips may be attached to the first side of the diaper fastening tab by means of thermal bonding or thermal welding, for example ultrasonic bonding, hot air bonding or laser welding. The advantage of these methods would be that no adhesive is required for attaching the strips to the diaper fastening tab while still providing easy and reliable attachment of the strips.

In one embodiment, the first and the at least one second strips of mechanical fastening elements are spaced from each other and the adhesive layer is thereby exposed between the strips of mechanical fastening elements such that an exposed adhesive layer is formed between the strips. The adhesive layer may also be surrounding the strips, i. e. left and right of the strips. With such a spaced configuration of the strips, both the mechanical fastener as well as the adhesive layer contributes to the hold down of the diaper fastening tab initially, i. e. before the use of the diaper fastening tab, as well as during use, i. e. when the diaper fastening tab is fastening and the mechanical fastening elements are engaging with a female fastening material of the diaper. In other words, a combination effect of a mechanical fastener and an adhesive fastener formed by the exposed adhesive layer is achieved, which further helps to increase peel strength of the diaper fastening tab. Alternatively, the adhesive layer for attaching the strips to the first side of the diaper fastening tab may only be present under the strips such that no exposed adhesive layer is formed even if the strips are spaced from each other. In other words, with such configuration, only the first major side of the diaper fastening tab is exposed between the strips and not an adhesive layer. Such a configuration may help to save adhesive material and may help to increase the flexibility of the diaper fastening tab.

In one embodiment, the first and the at least one second strips have a width extension in cross-direction of the diaper fastening tab of the diaper fastening tab, wherein the width of the first strip is lower than the width of the second strip. Such an arrangement provides for a cost-efficient solution as the material of the first strip made of a material with higher peel performance may be more expensive compared to the material of the second strip.

In one embodiment, the first and the at least one second strips have a width extension in cross-direction of the diaper fastening tab, wherein the strips have an equal width. Such a configuration provides for an easy to manufacture solution with an equal distribution of the fastening material.

In one embodiment, the height of the mechanical fastening elements of the first strip and the height of the mechanical fastening elements of the at least one second strip differ by at least 15 %. Such a difference in the height of the mechanical fastening elements may provide for a good balance of sufficient peel strength for the first strip and cost-efficiency for the second strip.

In one embodiment, the thickness of the backings of the first strip differs from the thickness of the backing of the at least one second strip by at least 15 %. Such a difference in the thickness of the backings of the strips of mechanical fastening elements may provide for a good balance of sufficient peel strength for the first strip and cost-efficiency for the second strip.

In one embodiment, the mechanical fastening elements of the first strip exhibit a height of 250 to 500 µm, preferably 300 to 400 µm. Such mechanical fastening elements may provide for a sufficient peel strength.

In one embodiment, the fastening elements of the at least one second strip exhibit a height of 100 to 225 µm, preferably 150 to 200 µm. Such mechanical fastening elements may provide for cost-efficiency of the mechanical fastening elements.

In one embodiment, the backing of the first strip exhibits a thickness of 80 to 150 µm, preferably 90 to 110 µm. Such mechanical fastening elements may provide for a sufficient peel strength.

In one embodiment, the backing of the at least one second strip exhibits a thickness of 10 to 70 µm, preferably 20 to 60 µm. Such mechanical fastening elements may provide for cost-efficiency of the mechanical fastening elements. The present disclosure further relates to a roll of diaper fastening tape. The roll comprises a plurality of diaper fastening tabs according to the present disclosure. The diaper fastening tabs are arranged in an endless form along a machine direction on the tape so that individual diaper fastening tabs can be provided from the diaper fastening tape by cutting the tape along its cross-direction. A roll of diaper fastening tape is beneficial as a roll provides for an easy and efficient method of storing the manufactured diaper fastening tape before use, e. g. in manufacturing. Also, for further handling like e. g. diaper manufacture, unwinding a roll of diaper fastening tape is an easy and reliable way of supplying diaper fasting tape to the manufacturing process.

The present disclosure moreover relates to a disposable diaper. The diaper has an outer edge defining the shape of the diaper comprising two opposite longitudinal edges. The diaper further comprises a diaper fastening tab according to the present disclosure. The diaper fastening tab is attached with its manufacturer's end to at least one longitudinal edge of the diaper. A diaper with such a diaper fastening tab is beneficial as the diaper fastening tab provides for reliable and cost-efficient prevention of unintentional disengagement of the mechanical fastener. Thus, the diaper is equipped with a reliable fastening system. The diaper may also provide more than one diaper fastening tab, for example two may be arranged on the diaper. If two diaper fastening tabs are provided it may be beneficial if each one is attached to an opposite longitudinal edge such that both are arranged at the same height.

The present disclosure moreover relates to a method of making a diaper fastening tab according to the present disclosure. The method comprises the steps of providing a carrier having a user's end and a manufacturer's end, a first and second major surface, a user's end portion adjacent to the user's end and a manufacturer's end portion adjacent to the manufacturer's end, providing a first strip of mechanical fastening elements; providing at least one second strip of mechanical fastening elements; attaching the first and the at least one second strips of mechanical fastening elements to the user's end portion of the carrier on a first major surface thereof such that the first strip is arranged towards the user's end of the diaper fastening tab and such that the at least one second strip is arranged towards the manufacturer's end of the substrate. The first strip of mechanical fastening elements has a higher peel strength as measured according to ASTM D 5170-98 than the at least one second strip of mechanical fastening elements. Such a method is beneficial as it provides for a reliable and cost-efficient way of manufacturing a diaper fastening tab with improved prevention of unintentional disengagement of the mechanical fastener.

In one embodiment, in the method of making a diaper fastening tab, the first and the at least one second strips comprise a separate backing from which the mechanical fastening elements protrude. Such a method is beneficial as the material of the first strip and the second strip can be manufactured separately and can then be applied by unwinding the two materials from different supply rolls.

The present disclosure moreover relates to a method of making a diaper according to the present disclosure with a diaper fastening tab according to the present disclosure. The method comprises the steps of providing a diaper having an outer edge defining the shape of the diaper comprising two opposite longitudinal edges, attaching at least one diaper fastening tab according to the present disclosure with its manufacturer's end portion to at least one longitudinal edge of the diaper, optionally folding the diaper fastening tab with its user's end around the longitudinal edge of the diaper. Such a method is beneficial as it provides for a reliable and cost-efficient way of manufacturing a diaper with a diaper fastening tab with improved prevention of unintentional disengagement of the mechanical fastener.

The diaper fastening tab may further comprise a fingerlift in the user's end portion. Typically, the fingerlift is arranged at or proximate the user's end of the diaper fastening tab. The fingerlift may comprise a film attached to the first major side of the diaper fastening tab. Alternatively, a thickened portion is arranged as fingerlift. Typically, the fingerlift is free of adhesive and/or free of mechanical fastening elements. The fingerlift is intended to by gripped by a user of the diaper and the diaper fastening tab attached thereto, respectively. The fingerlift may help to ease gripping of the diaper fastening tab.

It is to be understood that the above-mentioned embodiments all can be combined with each other.

The mechanical fastening elements of the strips preferably are integral with the exposed major surface of the backing of the strips. It is also possible that the mechanical fastening elements are bonded individually to the exposed major surface of the backing of the strips. Bonding of such individual mechanical fastening elements or strips of mechanical fastening elements, respectively, can be effected, for example, by adhesive bonding, by ultrasonic bonding, by thermal bonding or by stitching. It is disclosed, for example, in WO 00/50,229 to apply discrete hook strips to the exposed surface of a backing.

Mechanical fastening elements suitable in the present disclosure can be manufactured from a wide range of materials including thermoplastic polymers such as, for example, nylon, polyester, polyolefins or any combination of these. The mechanical fastening elements preferably comprise the material of which the backing is formed. The dimensions of the individual mechanical fastening elements can be varied widely depending on the application and the structure and loftiness of the complementary female fibrous material. When employing the diaper fastening tab of the present disclosure, for example, in disposable sanitary articles such as incontinence articles, diapers or napkins, the mechanical fastening elements comprising stems and, optionally, an enlarged section at the end of the stem opposite to major surface, preferably are between 40 µm and 2mm in height above the backing. The stems preferably have a cross-section with a maximum extension of between 10 µm and 250 µm. The ratio of the maximum extension of the enlarged portions of the mechanical fastening, elements at the end of the stems opposite to the exposed major surface of the backing, over the maximum extension of the cross-sections of the stems preferably is between 1.5:1 and 5:1.

The average surface density of the mechanical fastening elements with respect to the total area of the strips may vary broadly and preferably is between 10/cm² and 5,000/cm², more preferably between 20/cm² and 4,000/cm² and especially preferably between 25/cm² and 3,500/cm². If the density of the mechanical fastening elements is less than 10/cm² the strength of the mechanical bonding mechanism between the strips and a fibrous material brought into contact with the diaper fastening tab, tends to be insufficient for practical purposes. If the density of the mechanical fastening elements is above 5,000/cm², the single fastening elements tend to be very small and may not mechanically engage with the fibrous material to a sufficient and/or desirable extent. The manufacture of mechanical fastening elements which are suitable in the present disclosure is disclosed in the state of the art.

A mushroom-type hook web including a homogenous backing of thermoplastic resin and, integral with the backing, an array of upstanding stems projecting from the surface of the backing and having a mushroom head at the end of the stem opposite to the surface of the backing, is disclosed, for example, in US 5,077,870. This mushroom-type hook strip can be obtained by feeding the molten thermoplastic resin through a die to a rotating cylindrical mold which has cavities that are negatives of the upstanding stems. The molten resin is injected into the cavities in an excess of an amount that would fill the cavities so that a backing is formed. The resin is solidified and then stripped from the mold as a web that has an array of upstanding stems. The web is then passed between two calendar rolls whereby the roll contacting the tip of the stems is heated to allow for formation of the mushroom heads. US 5,679,302 discloses another mushroom-type hook strip where the enlarged portion at the end of the stems is essentially disc-shaped.

Mechanical fastener webs comprising a homogenous backing and, integral with the backing, an array of mechanical fastening elements whereby the enlarged portions have a variety of shapes, is disclosed, for example, in US 4,894,060.

The mechanical fastener webs and the specific geometry of the individual fastening elements disclosed in US 5,077,870, US 5,679,302 and US 4,894,060 are described here only by WO 2005/000180 PCT/US2004/014617 way of example and are not intended to limit the invention in any way. Other non-limiting examples of suitable mechanical fastener webs are described, for example, in US 4,984,339 and US 5,781,969.

The backing may be subjected to a mono-axial or biaxial stretching prior to cutting of the backing resulting in discrete portions of the backing. Biaxial stretching can be applied to the backing subsequently or simultaneously in CD and MD. The term stretch ratio as used above and below denotes the ratio of a linear dimension of a given portion of the stretched backing of the strips or the diaper fastening tab, respectively, to the linear dimension of the same portion of the backing of the strips or the diaper fastening tab, respectively, prior to stretching. The stretch ratios in MD and CD preferably are independently from each other between 1.1:1 and 8:1 and more preferably between 1.1:1 and 5:1. Biaxial stretching is preferred. Monoaxial stretching or sequential biaxial stretching can be performed, for example, by propelling a continuous web of the backing in the respective direction over rollers of increasing speed. Simultaneous biaxial stretching can be performed, for example, by using a flat film tenter apparatus as is described, for example, in US 4,675,582, US 4,825,111, US 4,853,602, US 5,036,262, US 5,051,225 and US 5,072,493.

The strips of mechanical fastening elements may exhibit various shapes such as, for example, circular, rectangular, triangular, essentially trapezoid or more complicated regular or irregular shapes. The strips can be obtained, for example, by passing a continuous web of a backing through an appropriately designed rotary knife cutter. The rectangular strips of the backing are arranged on the adhesive layer in a parallel fashion along the machine direction MD and in a distance in the cross-direction CD from each other so that an alternating sequence of parallel strips of exposed adhesive layer and of the discrete portions of the backing is obtained.

### Tested Mechanical Fastening Materials

### First mechanical fastening material

Strips of mechanical fastening elements comprises a mushroom-type hook web which is commercially available under the trade designation 3M™ Microreplicated hooks CHK-0732 from 3M Company, St. Paul, Minnesota, USA. The hook cap (head) geometry is elliptical with the large diameter in cross direction, wherein cross direction means a direction perpendicular to the direction in which the hook itself is manufactured. The density of the hooks is 248 hooks/cm² (1600/in²). This type of hook web is described in more detail in US-A-6,000,106, particularly in connection with Figure 9 thereof, and US-A-6,132,660, particularly Figure 6B thereof. The material further has a backing with a thickness of 100 µm and the hooks have a hook height 350 µm.

### Second mechanical fastening material

Strips of mechanical fastening elements comprises a mushroom-type hook web which is commercially available under the trade designation 3M™ Microreplicated HV hooks from 3M Company, St. Paul, Minnesota, USA of the type as mentioned above, except that the backing thickness and the hook geometry is different. The backing has a thickness of 50 µm and the hooks have a hook height of 190 µm. The hook density is the same as mentioned above (248/cm² or 1600/in²).

### Tested fibrous materials

The following test fibrous materials were used as typical examples for undergarment materials in order to test the 90° peel strength of the fastening elements used in accordance with the invention:

### First fibrous material

A fibrous, nonwoven female fastening material of the type CLP-06955 NWLZ, (available from 3M Company, 3M Center, St. Paul, MN55144-1000, USA)

### Second fibrous material

A female fastening material comprising TRM 898 & EBL (both available from 3M Company, 3M Center, St. Paul, MN55144-1000, USA)

### Examples

In order to demonstrate the advantageous effects of the fastening tabs according to the present disclosure, several tests have been conducted.

### Peel test ASTM D 5170-98: 90° peel strength

The 90° peel strength was measured according to ASTM D 5170-98 using a roll down weight of 5000 g. Instead of removing the hook from the fabric just once as described in the above test method this was done twice prior to testing. The tested hook area was 25.4 mm x 15 mm, wherein the 25.4 mm was used as the peel front. The maximum peel force in N/25.4 mm is reported in Table 1 below for several test fabrics.

### Sample Preparation

Hook and fabric material were cut out in a width of 1 inch (25.4 mm). The hook strip was placed onto the fabric and pushed down by a flat steel plate for a period of two seconds. The steel plate was carefully removed and the test specimen was rolled down with a 5 kg roller with 5 cycles. This initial closure was opened by hand and again fastened and opened using the same procedure as described. After the third fastening of the hook into the fabric using 5 cycles of a 5 kg roll down weight the test specimen was positioned into the clamps of the tensile tester being 1 inch (25.4 mm) apart. The testing speed was set to 300 mm/min and the peak force was recorded along a travel distance of the clamps of 50 mm.

### Example 1:

Discrete strips of a first and second fastening material were obtained from a mushroom-type hook web which is commercially available under the trade designation 3M™ Microreplicated hooks CHK00732 and HV hooks, both from 3M Company, St. Paul, MN, USA, by cutting. The strips had the dimension of 6,5 mm in CD and of 40 mm in MD.

The average height of the mechanical fastening elements of the type CHK00732 was about 350 µm and of the type HV Hooks was 190 µm.

The first and second strips of mechanical fastening elements were attached to the user's end portion of the carrier forming the diaper fastening tab such that the first strip is located towards the user's end and such that the second strip is located towards the manufacturer's end.

The thickness of the backing of the strips of mechanical fastening elements was 100 µm for the CHK00732 material and 50 µm for the HV Hooks material.

The mechanical fastening elements were integral with the backing of the strips, and they were distributed essentially homogenously over the surface area of the hook web at a density of 252 hooks/cm² (1,626 hooks/in²) for both types, CHK00732 and HV hooks.

The single mechanical fastening elements had a stem with a diameter of about 250 µm and an enlarged, oval-shaped portion at the end of the stems opposite to the backing of the mechanical fastening material web for both types, CHK00732 and HV hooks. The discrete portions of the mechanical fastening material were then applied onto a PP film substrate forming the carrier and bearing a polystyrene-polyisoprene block-copolymer pressure-sensitive adhesive layer with a thickness of about 35 µm in a parallel fashion along the MD and in a distance of 1 mm between adjacent strips in the CD so that an alternating sequence of parallel strips of exposed adhesive layer and of the two discrete strips of the mechanical fastening elements was obtained.

### Comparative example 1:

Comparative example 1 was made as described under example 1, except that there were two strips of the same mechanical fastening material of the type CHK00732 hooks, i. e. the first mechanical fastening material.

### Comparative example 2:

Comparative example 1 was made as described under example 1, except that there were two strips of the same mechanical fastening material of the type HV hooks, i. e. the second mechanical fastening material.

### Test results

**Table 1: Test Results Peel Strength with first fibrous material**

| Test no. | Example | Hook mat. | Fibrous material | Peel strength [N/25,4mm] |
|---|---|---|---|---|
| | | | | |
| 1 | 1 | 732+HV | First | 3,77 |
| 2 | C1 | 732+732 | First | 3,93 |
| 3 | C2 | HV+HV | First | 3,23 |

From Table 1, it is apparent, that the Example 1 has an almost similar peel strength value compared to that of Comparative Example C2 tested on the first fibrous material.

Although Example 1 comprises partially a lower performing hook material (HV Hook), it performs with respect to peel strength almost similar to the example with complete higher performing hooks (C1), but better than the Comparative example with complete lower performing hooks (C2), when engaging with the first fibrous material.

**Table 2: Test Results Peel Strength with first fibrous material**

| Test no. | Example | Hook mat. | Fibrous material | Peel strength [N/25,4mm] |
|---|---|---|---|---|
| 6 | 1 | 732+HV | Second | 8,51 |
| 9 | C1 | 732+732 | Second | 7,60 |
| 10 | C2 | HV+HV | Second | 5,21 |

From Table 2, it is apparent, that the Example 1 has a slightly higher peel strength value than that of Comparative Example C1 tested on the second fibrous material and is much higher than that of Comparative example C2.

Although Example 1 comprises partially a lower performing hook material (HV Hook), it performs with respect to peel strength slightly higher than the example with complete higher performing hooks (C1) and much better than the Comparative example with complete lower performing hooks (C2).

The invention will now be described in more detail with reference to the following Figures exemplifying particular embodiments of the invention:
Fig. 1 is a top view of a fastening tab according to the present disclosure.
Fig. 2 is a cross-sectional side view of the fastening tab of Fig. 1 along the line A-A.
Fig. 3 is a perspective view of a roll of diaper fastening tape comprising a plurality of diaper fastening tabs according to the present disclosure.
Fig. 4 is a schematic, perspective view of a diaper with a fastening tab according to the present disclosure.

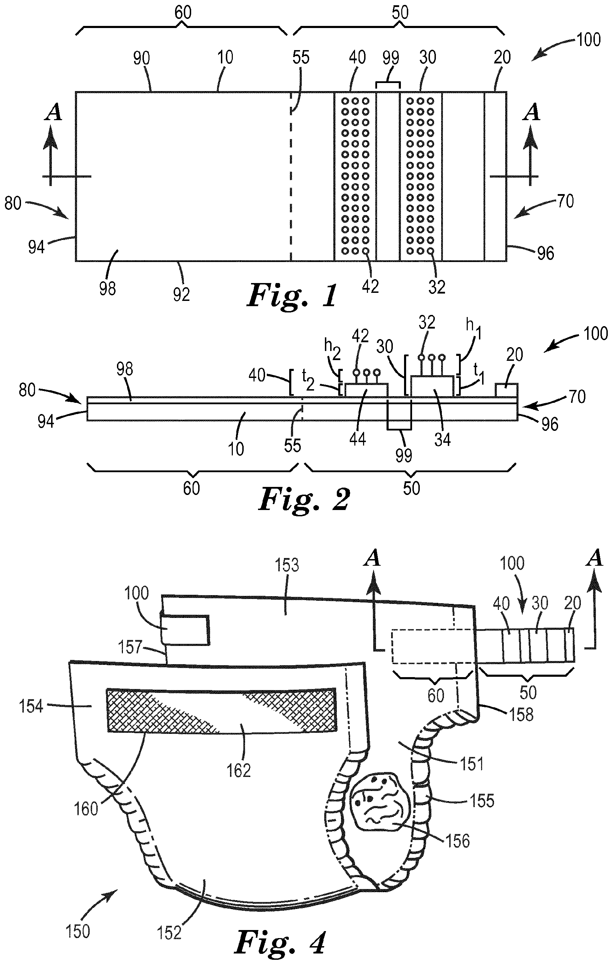

As shown in Fig. 1, the diaper fastening tab 100 is in the form of an elongate strip, having two opposing longitudinal sides 90, 92 and two opposing transverse sides 94, 96, formed from a carrier 10. In the embodiment shown in Fig. 1, the longitudinal sides 90, 92 on the one hand and the transverse sides 94, 96 on the other hand are parallel to each other. The diaper fastening tab 100 includes a first and a second strip 30, 40 of mechanical fastening elements 32, 42 arranged on a first major side of the diaper fastening tab 100. The strips 30, 40 typically extend in machine direction between the longitudinal side edges 90, 92 of the carrier 10. The strips 30, 40 each further comprise a backing 34, 44, from which the mechanical fastening elements 32, 42 protrude. The diaper fastening tab 100 is provided with a user's end 70 to be gripped by a user and a manufacturer's end 80 for attaching the diaper fastening tab 100 to a diaper (not shown, see Fig. 4). The term "end" denotes an edge of the diaper fastening tab, whereas the term "portion" denotes an area on the first surface of the diaper fastening tab. The diaper fastening tab 100 further comprises a user's end portion 50 adjacent the user's end 70 and a manufacturer's end portion 60 adjacent the manufacturer's end 80. The user's end portion 50 and the manufacturer's end portion 60 are separated by a (hypothetical) separation line 55 which is shown for illustrative purpose only.

The first strip 30 is located in the user's end portion 50 of the carrier 10, generally towards the user's end 70 of the diaper fastening tab 100. The second strip 40 is located in the user's end portion 50 of the carrier 10, generally towards the manufacturer's end 80 of the diaper fastening tab 100, but still within the user's end portion 50. There may be more than two strips arranged on the user's end portion 50. The strips 30, 40 may be mounted to the carrier 10 by means of an adhesive layer 98, i. e. the side of backing of the strips 30, 40 facing towards the carrier 10 are typically covered by an adhesive layer 98. The extension of the adhesive layer 98 in cross-direction may be greater than that of the strips 30, 40, i. e. portions of the user's end 50 not covered by the strips 30, 40 may be covered by an adhesive layer 98. The adhesive layer 98 from the user's end portion 50 may also extend partially or entirely into the manufacturer's end portion 60. In case that the adhesive fully covers the manufacturer's end portion 60 as well, the adhesive layer 98 may also be used to mount the diaper fastening tab 100 to the diaper 150 (not shown here, see Fig. 4).

As shown in Figs. 1 and 2, strips 30, 40 are discrete strips and are spaced from each other. An adhesive layer 98 present in the user's end portion 50 may be exposed that the region between the strips 30, 40 and/or surrounding the strips 30, 40, i. e. left and right of the strips 30, 40, such that an exposed adhesive layer 99 is formed between the strips 30, 40. It is also conceivable that the strips 30, 40 abut each other. In this case, an adhesive underneath of the strips 30, 40 may only be exposed to a small extend or not at all. The fastening tab 100 further comprises a fingerlift formed by a film attached to the first major surface of the carrier 10 of the diaper fastening tab 100. The fingerlift 20 is arranged proximate to or abutting the user's end 70. The fingerlift 20 is provided to ease gripping of the user's end portion 50 of the diaper fastening tab 100, for example for fastening or unfastening of the diaper fastening tab 100 when the diaper 150 (not shown here, see Fig. 4) is in use.

Fig. 2 shows the diaper fastening tab 100 is a cross-sectional side view. As can be seen from Fig. 2, the thickness of the backing 34, 44 is indicated with t1, t2. The height of the mechanical fastening elements 32, 42 is indicated with h1, h2. In the embodiment shown in Fig. 2, the backing 34 of the first strip 30 has a greater thickness t1 than the thickness t2 of the backing 44 of the second strip 40. Also, in this embodiment, the height h1 of the mechanical fastening elements 32 of the first strip 30 is greater than the height h2 of the mechanical fastening elements 42 of the second strip 40.

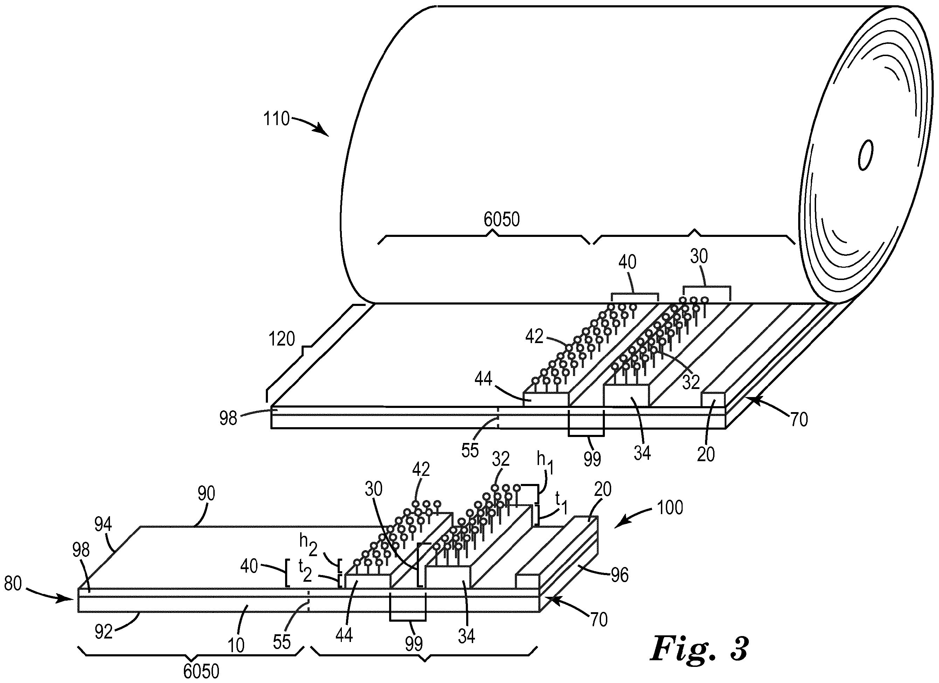

Fig. 3 shows in a perspective view a roll 110 of diaper fastening tape 120 and a diaper fastening tab 100 obtained therefrom. Diaper fastening tab 100 was cut from the diaper fastening tape 120 after unwinding of the roll 110 of diaper fastening tape 120. As mentioned above, the diaper fastening tab 100 as well as the diaper fastening tape 120 comprises a carrier 10, a first and a second strip 30, 40 of mechanical fastening elements 32, 42 protruding from the backing 34, 44 of the strips 30, 40. The strips 30, 40 extend in machine direction of the diaper fastening tape 120, i. e. the direction of unwinding the diaper fastening tape 120. The diaper fastening tab 100 and the diaper fastening tape 120 comprises a user's end 70 and a manufacturer's end 80. The diaper fastening tab 100 as well as the diaper fastening tape 120 further comprises a user's end portion 50 adjacent the user's end 70 and a manufacturer's end portion 60 adjacent the manufacturer's end 80. The user's end portion 50 and the manufacturer's end portion 60 are separated by a (hypothetical) separation line 55 which is shown for illustrative purpose only. The diaper fastening tab 100 as well as the diaper fastening tape 120 further comprises a fingerlift 20 arranged in the user's end portion 50 close to or adjacent the user's end 70 of the diaper fastening tab 100 or the diaper fastening tape 120, respectively. Strips 30, 40 may be attached to the first major side of the carrier of the diaper fastening tab 100 or the diaper fastening tape 120, respectively, by means of an adhesive (not shown here).

A schematic, perspective view of one embodiment of an absorbent article 150 having a diaper fastening tab 100 according to the present disclosure is shown in FIG. 4. Absorbent article 150 includes a topsheet 151 and a backsheet 152 forming the diaper chassis. The chassis also has first and second opposing longitudinal edges 157, 158 extending from a rear waist region 153 to an opposing front waist region 154. The term "longitudinal" refers to the length of the absorbent article 150 from the rear waist region 153 towards the front waist region 154, for example, when it is in an open configuration.

At least one of the front waist region 154 or the rear waist region 153, more typically the rear waist region 153, comprises at least one fastening tab 100 as it is shown in Fig. 4. In the embodiment shown in Fig. 4, the diaper 150 comprises a diaper fastening tab 100 attached to each longitudinal edge 157, 158 of the diaper 150 in the rear waist region 153. The diaper fastening tab 100 includes a carrier 10 and first and second strips 30, 40 of mechanical fastening elements 32, 34. The first strip 30 is located in the user's end portion 50 of the first major surface of the carrier 10, generally towards the edge of the user's end 70 of the fastening tab 100. The second strip 40 is located in the user's end portion 50 of the first major surface of the carrier 10, generally towards the edge of the manufacturer's end 80 of the fastening tab 100, but still within the user's end portion 50. The diaper 150 comprises an absorbent core 156 between the topsheet 151 and the backsheet 152. The diaper 150 further comprises leg elastics 155 arranged at least partially at the longitudinal sides of the diaper 150. Moreover, the diaper 150 comprises a landing zone 160 with a female fastening material 162. The landing zone 160 and the female fastening material 162, respectively, are configured and arranged to engage with the mechanical fastening elements 32, 42 of the diaper fastening tab 100, when fastening or closing the diaper 150.

## Claims

1. A diaper fastening tab (100) comprising:
- a user's end (70) to be gripped by a user,
- an opposite manufacturer's end (80),
- a first and a second major surface,
- a user's end portion (50) adjacent to the user's end (70),
- a manufacturer's end portion (60) adjacent to the manufacturer's end (80),
wherein the user's end portion (50) comprises on the first major surface a first and at least a second strip (30, 40) of mechanical fastening elements (32, 42) each with a different peel strength as measured according to ASTM D 5170-98 and wherein the first strip (30) with a higher peel strength is located towards the user's end (70) and the second strip (40) with a lower peel strength is located towards the manufacturer's end (80).

2. The diaper fastening tab (100) according to claim 1, wherein the manufacturer's end portion (60) is free of mechanical fastening elements (32, 42).

3. The diaper fastening tab (100) according to claim 1 or 2, wherein each of the first and at the least one second strips (30, 40) of mechanical fastening elements (32, 42) comprises a separate backing (34, 44) from which the mechanical fastening elements (32, 42) protrude.

4. The diaper fastening tab (100) according to claim 3, wherein the first strip (30) with a higher peel strength comprises mechanical fastening elements (32) exhibiting a larger height (h1) compared to the height (h2) of the mechanical fastening elements (42) of the at least one second strip (40) with a lower peel strength and/or wherein the first strip (30) with a higher peel strength comprises a backing (34) exhibiting a greater thickness (t1) compared to the thickness (t2) of the backing (44) of the at least one second strip (40) with a lower peel strength.

5. The diaper fastening (100) tab according to claim 3 or 4, wherein the mechanical fastening elements (32, 42) comprise a stem projecting from a major surface of the backing (34, 44) with an enlarged section which is positioned at the end of the stem opposite of the surface of the backing (34, 44).

6. The diaper fastening tab (100) according to any of the preceding claims, wherein the mechanical fastening elements (32, 42) are uniformly shaped within one strip.

7. The diaper fastening tab (100) according to any of the preceding claims, wherein the first and the at least one second strips (30, 40) are attached to the first major side of the fastening tab by an adhesive layer (98).

8. The diaper fastening tab (100) according to claim 7, wherein the first and the at least one second strips (30, 40) of mechanical fastening elements (32, 42) are spaced from each other and the adhesive layer (98) is thereby exposed between the strips of mechanical fastening elements such that an exposed adhesive layer (99) is formed between the strips (30, 40).

9. The diaper fastening tab (100) according to any of the preceding claims, wherein the first and the at least one second strips (30, 40) have a width extension in cross-direction of the diaper fastening tab (100) and the width of the first strip (30) is lower than the width of the second strip (40).

10. The diaper fastening tab (100) according to any of the claims 3 to 9, wherein the height (h1) of the mechanical fastening elements (32) of the first strip (30 and the height (h2) of the mechanical fastening elements (42) of the at least one second strip (40) differ by at least 15 % and/or wherein the thickness (t1) of the backing (34) of the first strip (30) differs from the thickness (t2) of the backing (44) of the at least one second strip (40) by at least 15 %.

11. A roll (110) of diaper fastening tape (120) comprising a plurality of diaper fastening tabs (100) according to any of the claims 1 to 10 wherein the diaper fastening tabs (100) are arranged in an endless form along a machine direction on the tape (120) so that individual diaper fastening tabs (100) can be provided from the diaper fastening tape (120) by cutting the tape (120) along its cross-direction.

12. A disposable diaper (150), said diaper having an outer edge defining the shape of the diaper (150) comprising two opposite longitudinal edges (157, 158), the diaper (150) further comprising a diaper fastening tab (100) according to any of the claims 1 to 10, the diaper fastening tab (100) being attached with its manufacturer's end (60) to at least one longitudinal edge (157, 158) of the diaper (150).

13. Method of making a diaper fastening tab (100) according to any of claims 1 to 10, the method comprising the steps of
- providing a carrier having a user's end (70) and a manufacturer's end (80), a first and second major surface, a user's end portion (50) adjacent to the user's end (70) and a manufacturer's end portion (60) adjacent to the manufacturer's end (80),
- providing a first strip (30) of mechanical fastening elements (32);
- providing at least one second strip (40) of mechanical fastening elements (42);
- attaching the first and the at least one second strips (30, 40) of mechanical fastening elements (32, 42) to the user's end portion (50) of the substrate on a first major surface thereof such that the first strip (30) is arranged towards the user's end (70) of the diaper fastening tab (100) and such that the at least one second strip (40) is arranged towards the manufacturer's end (80) of the substrate,
wherein a first strip (30) of mechanical fastening elements (32) has a higher peel strength as measured according to ASTM D 5170-98 than the at least one second strip (40) of mechanical fastening elements (42).

14. Method according to claim 13, wherein each of the first and the at least one second strips (30, 40) comprise a separate backing (34, 44) from which the mechanical fastening elements (32, 42) protrude.

15. Method of making a diaper (150) according to claim 14 with a fastening tab (100) according to any of claims 1 to 10, the method comprising the steps of
- providing a diaper (150) having an outer edge defining the shape of the diaper (150) comprising two opposite longitudinal edges (157, 158),
- attaching at least one diaper fastening tab (100) according to any one of claims 1 to 10 with its manufacturer's end portion (60) to at least one longitudinal edge (157, 158) of the diaper (150),
- optionally folding the diaper fastening tab (100) with its user's end (70) around the longitudinal edge (157, 158) of the diaper (150).
